# EUROPEAN PATENT SPECIFICATION

(11) **EP 4 301 249 B1**
(45) Date of publication and mention of the grant of the patent: **11.11.2026**
(21) Application number: 22710185.4
(22) Date of filing: 25.02.2022
(51) Int. Cl.: A61B 17/16, B23D 73/00

(54) **SURGICAL TOOL AND CORRESPONDING PRODUCTION METHOD**
CHIRURGISCHES WERKZEUG UND ZUGEHÖRIGES HERSTELLUNGSVERFAHREN
OUTIL CHIRURGICAL ET PROCÉDÉ DE PRODUCTION CORRESPONDANT

(30) Priority: 02.03.2021 IT 202100004808
(43) Date of publication of application: 10.01.2024
(73) Proprietor: HPF S.R.L., 33030 Forgaria nel Friuli (UD) (IT)
(72) Inventor: LUALDI, Gabriele, 33034 FAGAGNA (IT); DE AGOSTINI, Pablo, 33040 MOIMACCO (IT)
(74) Representative: Petraz, Gilberto Luigi
(86) International application number: PCT/IT2022/050035
(87) International publication number: WO 2022/185351

(56) References cited:
- BE-A3- 1 015 812
- US-A- 3 412 733
- US-A- 5 006 121
- US-A1- 2010 262 146

## Description

### FIELD OF THE INVENTION

The present invention concerns a surgical tool, in particular for prosthetic surgery, suitable for the removal and/or compaction of bone tissues in the context of surgical interventions for the implantation of orthopedic prostheses, as well as the method to produce it. The surgical tool of the present invention is applied in the preparation of any bone canal for the implantation of a shaped stem of a prosthesis such as, for example, but without limitations in general, a femoral prosthesis, a prosthesis of the humerus, tibia or shoulder, and others.

### BACKGROUND OF THE INVENTION

Surgical tools are known, such as for example broaches or rasps, used for the preparation of a bone canal or seating, for the implantation of orthopedic prostheses, for example the stem of a femoral prosthesis, a prosthesis of the humerus, the tibia, the shoulder and others.

This type of surgical tool normally has an elongated shape, tapered and mating with that of the stem of the prosthesis to be implanted and, generally, they are forcibly inserted into a hole made in a portion of bone prepared in advance, so as to widen it and give it a desired shape, for example with a square or rectangular cross section.

Known surgical tools comprise at a first end a connector for a tool holder, at a second end a penetration tip and a cutting surface disposed between the two ends.

The shape of the cutting surface is different depending on the use of the tool.

In particular, the cutting surface of broaches is conformed as steps with edges that can be either sharp, or rounded or beveled, to act adequately on the bone tissue to remove it or to compact it.

On the contrary, the cutting surface of rasps comprises a plurality of small cutting elements conformed as punctiform protuberances that protrude from the lateral surface of the tool and the main function of which is to remove the bone tissue.

These known surgical tools are generally made in a single body, that is, without a break in continuity, substantially in two alternative ways: a) by forging, or b) by machining with removal of chip, usually using numerically controlled machine tools.

Furthermore, in both of these alternative methods, a grinding of the semifinished surgical tool can follow, in order to finish the cutting surface.

One disadvantage of known surgical tools is that they have rather high production costs.

In fact, in the case of forging, it is necessary to prepare a hammer and the corresponding dies, which require high initial investments, while machining with chip removal, in addition to a considerable initial investment for the machine tool, requires long machining times; therefore, in both cases, there is a high production cost for each surgical tool made.

In addition, another disadvantage is that both of these production methods are not very flexible in terms of customizing the surgical tool which instead, in practice, requires a great deal of customization.

Document US-A-2010/262146 describes tapered surgical reamers and broaches for preparing a medullary canal, said reamers and broaches being manufactured by assembling, around a tapered drive shaft, a multiplicity of plates comprising flutes or teeth, said plates having a taper corresponding to the taper of the respective reamer or broach.

Document US-A-5,006,121 describes a broach for the femur or other bones for preparing them for the purpose of implanting a prosthesis, for example a hip prosthesis.

Document US-A-3,412,733 discloses a reamer or miller for acetabular prostheses.

Document BE-A-1.015.812 describes a rasp for prosthetic surgery, in particular for implanting a prosthesis in the femur.

There is therefore a need to make a surgical tool, and to perfect corresponding new and original manufacturing methods, which can overcome at least one, better if all, of the disadvantages of the state of the art.

In particular, one purpose of the present invention is to provide a surgical tool which is economical to make, and which can be easily customized according to the needs of the end user, who is normally a surgeon, or a professional in the field of prosthetics.

Another purpose of the present invention is to perfect a method to produce a surgical tool which is quick, economical and flexible in terms of customization of the surgical tool.

The Applicant has devised, tested and embodied the present invention to overcome the shortcomings of the state of the art and to obtain these and other purposes and advantages.

### SUMMARY OF THE INVENTION

The present invention is set forth and characterized in the independent claims. The dependent claims describe other characteristics of the present invention or variants to the main inventive idea.

In accordance with the above purposes, a surgical tool for the preparation of a bone canal for the implant of the stem of a prosthesis comprises at least one cutting unit configured to remove and/or compact bone tissues.

According to one aspect of the present invention, the cutting unit comprises a plurality of autonomous cutting members independent from each other, disposed each one in contact with the adjacent one according to a determinate sequence along a common longitudinal axis.

According to another aspect of the present invention, the surgical tool also comprises a first element associated with the cutting unit and configured to be mechanically connected to an external tool holder.

According to another aspect of the present invention, each of the cutting members also comprises a central cavity substantially coaxial to the longitudinal axis and the plurality of central cavities of the cutting members disposed in sequence defines a central channel.

According to another aspect of the present invention, the surgical tool also comprises a central support member inserted inside the central channel and configured to give structural rigidity to the cutting unit.

According to another aspect of the present invention, the first element is disposed at a first end of the central support member and the surgical tool also comprises a second element disposed at a second end of the central support member, opposite the first end.

According to another aspect of the present invention, at least one of either the first element or the second element is made in a single body with the central support member.

In accordance with another aspect of the present invention, a method to produce a surgical tool comprises at least the following steps:
- producing, or having available, a plurality of cutting members, each one distinct and separate from the other;
- disposing the cutting members each one in contact with another adjacent one according to a determinate sequence along a common longitudinal axis in order to make up a cutting unit.

According to one aspect of the present invention, each of the cutting members is made with a central cavity and the plurality of central cavities defines a central channel of the cutting unit.

In these cases, the method of the present invention also comprises the following steps:
- producing, or having available, a central support member mating in shape with the central channel;
- inserting each of the cutting members on the central support member.

### BRIEF DESCRIPTION OF THE DRAWINGS

These and other aspects, characteristics and advantages of the present invention will become apparent from the following description of some embodiments, given as a non-restrictive example with reference to the attached drawings wherein:
- fig. 1 is a three-dimensional view of a surgical tool according to the present invention in accordance with a first embodiment;
- fig. 2 is a three-dimensional exploded view of the surgical tool according to fig. 1;
- fig. 3 is a three-dimensional view of a surgical tool according to the present invention in accordance with a second embodiment;
- fig. 4 is an exploded section view of the cutting unit of the surgical tool of fig. 1;
- fig. 5 is a section view of the cutting unit of the surgical tool of fig. 1;
- figs. from 6 to 8 are section views of different embodiments of the surgical tool according to the present invention;
- fig. 9 is a lateral view of the surgical tool of fig. 1;
- figs. from 10 to 13 are schematic views of different embodiments of the cutting members of the surgical tool according to the present invention.

We must clarify that in the present description and in the claims the term vertical, with its declinations, has the sole function of better illustrating the present invention with reference to the drawings and must not be in any way used to limit the scope of the present invention, or the field of protection defined by the attached claims. For example, by the term vertical we mean an axis or a plane that can be either perpendicular to the line of the horizon, or inclined, even by several degrees, for example up to 20°, with respect to the latter.

Furthermore, the people of skill in the art will recognize that certain sizes or characteristics in the drawings may have been enlarged, deformed, or shown in an unconventional or non-proportional way in order to provide a version of the present invention that is easier to understand. When sizes and/or values are specified in the following description, the sizes and/or values are provided for illustrative purposes only and must not be construed as limiting the scope of protection of the present invention, unless such sizes and/or values are present in the attached claims.

The phraseology and terminology used here are also solely for the purposes of providing non-limiting examples. Furthermore, all the intervals reported here shall be understood to include the extremes, including those that report an interval between two values, unless otherwise indicated.

To facilitate comprehension, the same reference numbers have been used, where possible, to identify identical common elements in the drawings. It is understood that elements and characteristics of one embodiment can be conveniently combined or incorporated into other embodiments without further clarifications.

### DESCRIPTION OF SOME EMBODIMENTS OF THE PRESENT INVETION

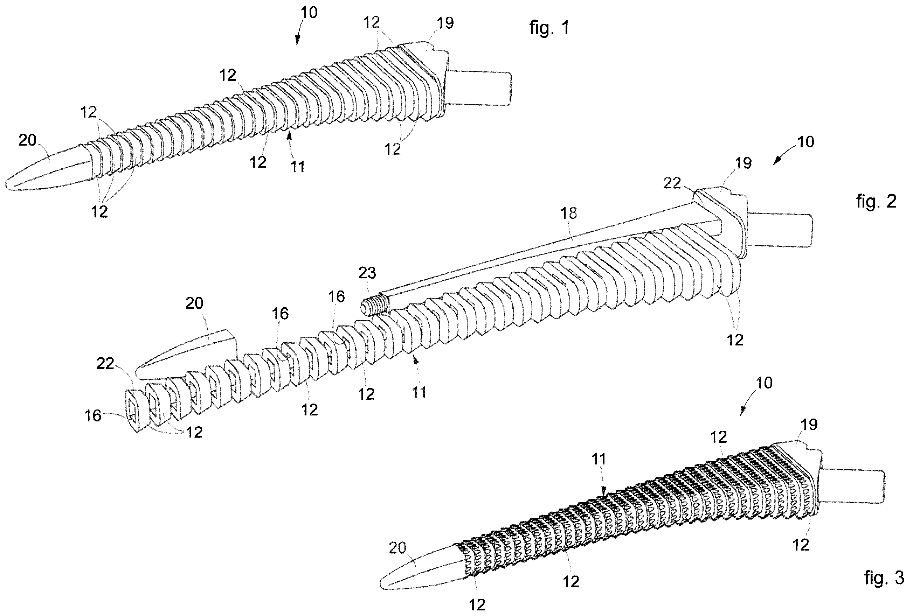

With reference to figs. 1, 2 and 3, the reference number 10 indicates a surgical tool according to some embodiments of the present invention.

The surgical tool 10 comprises a cutting unit 11 configured to remove and/or compact bone tissue for the preparation of a bone canal suitable to receive inside it the stem of an orthopedic prosthesis, such as for example, but not limited to, a femoral prosthesis, a prosthesis of the humerus, tibia, shoulder and others more.

We wish to clarify that in the present description and in the attached claims, with the expression "cutting", referring to the surgical tool 10 and/or its parts, we mean the function of removing and/or compacting bone tissue.

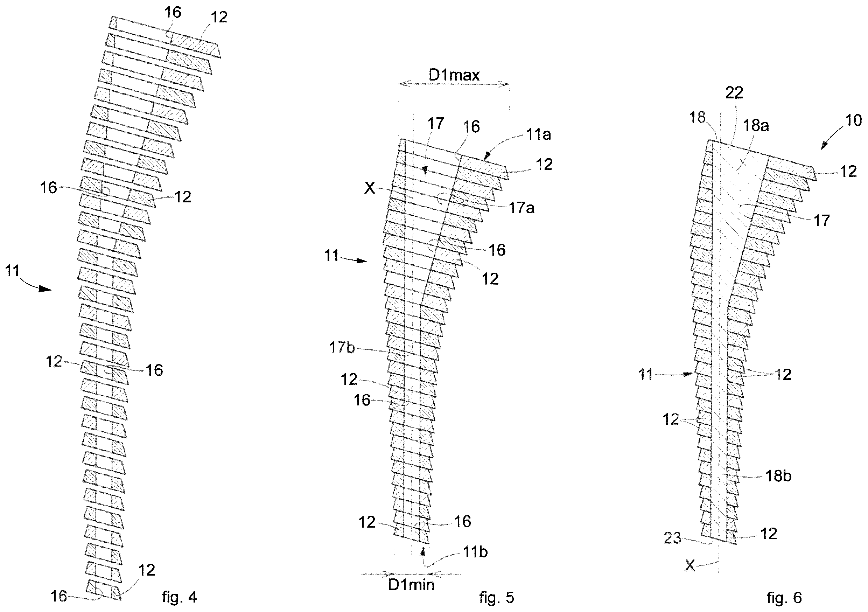

The cutting unit 11, as a whole, is oblong in shape and is substantially tapered along its longitudinal axis X from a first end 11a (fig. 5) to a second end 11b.

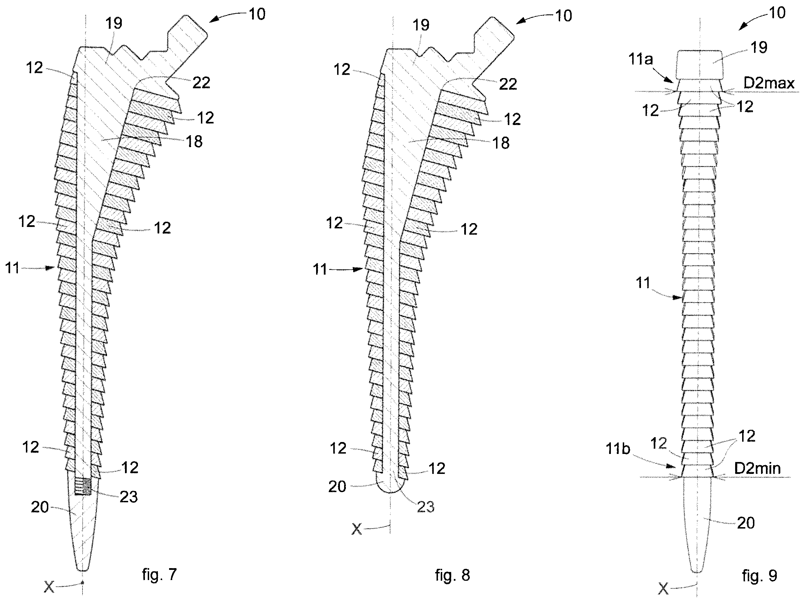

**In** particular, in the embodiments shown here, the cross-section of the cutting unit 11 is substantially rectangular and is defined by a first size D1 and by a second size D2 (fig. 9), perpendicular to the first size D1 (fig. 5). The first size D1 varies from a maximum value D1ₘₐₓ to a minimum value D1ₘᵢₙ, while the second size D2 (fig. 9) varies from a maximum value D2ₘₐₓ to a minimum value D2ₘᵢₙ.

The trend with which the first size D1 and the second size D2 of the cutting unit 11 vary progressively along the longitudinal axis X is a function of the desired shape to be given to the bone canal that will receive the stem of the prosthesis to be implanted.

According to one aspect of the present invention, the cutting unit 11 comprises a plurality of autonomous cutting members 12 independent from each other, which are each disposed each one in contact with the adjacent one according to a determinate sequence along the common longitudinal axis X.

We wish to clarify that the longitudinal axis X as above can be either a straight line or a curved line, or a combination of rectilinear and curved segments.

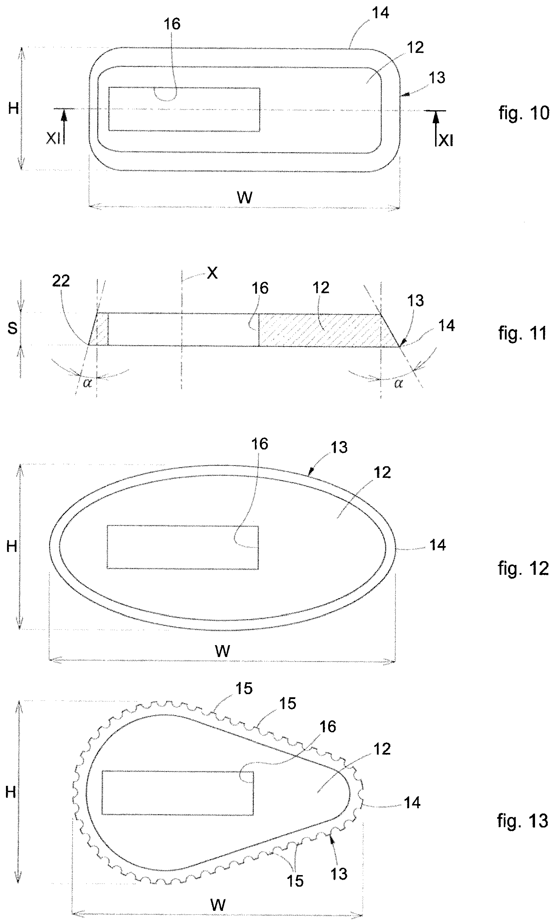

Each cutting member 12 in fact substantially comprises, or consists of, a metal plate with a quadrilateral shape (figs. 10 and 11), preferably made of steel, or titanium, having a determinate thickness S. The cutting members 12 can even have thicknesses S different from each other. Furthermore, it is not excluded that the cutting members 12 may also have other shapes, such as, for example, an elliptical, polygonal, or round shape and others, as exemplified in figs. 12 and 13.

Each cutting member 12, viewed in plan (figs. from 10 to 13) has a determinate height H and a determinate width W, which may not coincide with the first size D1 and second size D2.

In the embodiments described here, the cutting members 12 have the height H and the width W different from each other and the sequence in which they are disposed along the longitudinal axis X is such as to give the cutting unit 11 the desired tapered profile.

Furthermore, the lateral walls of each cutting member 12 are inclined in the direction of the thickness S by an angle α with respect to the longitudinal axis X. This angle α is variable according to the considered side of the cutting member 12 and it is comprised between about 10° and about 40°.

Some embodiments of the present invention can provide that the angle α can have other values, even negative, or that it can be substantially equal to zero.

Each of the cutting members 12 also comprises at least one cutting element 13.

In accordance with the embodiment shown in figs. 1, 10, 11 and 12, the cutting elements 13 of each cutting member 12 can comprise one or more lateral edges 14, each of which can be sharpened, or beveled, connected or rounded, so that the surgical tool 10 can be considered a broach.

In accordance with the embodiment shown in figs. 2 and 13, the cutting elements 13 comprise a plurality of protrusions 15 which are made on the lateral walls of each cutting member 12, or, alternatively, in correspondence with a lateral edge 14 (fig. 13) of the cutting member 12, so that the surgical tool 10 can be considered a rasp.

In one embodiment, not shown in the drawings but easily understood by a person of skill in the art, the cutting members 12 of the cutting unit 11 are solid, that is, without empty spaces inside them, and are mechanically connected to each other, for example by means of welding points or lines, or other types of suitable connection, for example by gluing.

In the embodiments described here, each cutting member 12 instead comprises a central cavity 16 (figs. from 3 to 8) which, in cooperation with the central cavities 16 of the cutting members 12, defines a central channel 17 (fig. 5) of the cutting unit 11.

The central cavity 16 of each cutting member 12 is mating in shape with the next or previous cutting member 12 along the longitudinal axis X, in order to give the central channel 17 a desired shape.

In particular, the central channel 17 comprises a first tapered portion 17a and a second rectilinear portion 17b; although the central channel 17 can have other shapes, for example a totally tapered shape, or a totally rectilinear shape or others.

Preferably, the surgical tool 10 also comprises a central support member 18 inserted in the central channel 17 and configured to give stability and strength to the cutting unit 11.

The central support member 18 is mating in shape with the central channel 17, preferably with a minimum clearance. In particular, in the embodiments shown here, the central support member 18 comprises a first tapered portion 18a and a second rectilinear portion 18b (fig. 6). The fact that the central channel 17 and the central support member 18 are mating in shape and that they comprise at least a first tapered portion is particularly advantageous, since it allows to verify that the cutting members 12 are disposed in the correct sequence.

The cross-section of the central cavities 16 and of the central support member 18 is, preferably but not limited to, quadrilateral, in such a way as to prevent rotation of the cutting members 12 around the longitudinal axis X (figs. 10, 12 and 13).

The surgical tool 10 also comprises a first element 19 associated with the cutting unit 11 and comprising a connector configured to be connected to an external tool holder.

In an embodiment not shown, the cutting member 12 at the first end 11a of the cutting unit 11 is mechanically connected, for example by welding or gluing, to the first element 19.

Instead, in the embodiments shown here that provide the presence of the central support member 18, the first element 19 is made in a single body with the latter, and it is disposed at a first end 22 thereof.

In addition, the surgical tool 10 also comprises a second element 20 connected to the central support member 18, in particular to a second end 23 thereof, opposite the first end 22. The second element 20, in cooperation with the first element 19, prevents the cutting unit 11 from sliding out of the central support member 18.

In particular, with reference to fig. 7, the second end 23 of the central support member 18 comprises a coupling portion configured to mechanically and reversibly connect to the second element 20. This conformation is particularly advantageous since it allows to disconnect the second element 20 from the central support member 18, allowing to replace the cutting unit 11, which can therefore be of the disposable type.

Alternatively, in possible embodiments, an example of which is shown in fig. 8, the second element 20 can be made in a single body with the central support member 18.

In other possible variants (not shown), the first element 19 can be mechanically and reversibly connected to the first end 22 of the central support member 18, for example by means of a threaded coupling.

It should be noted that even in these preferential embodiments which provide the presence of the central support member 18, the cutting members 12 can be connected mechanically, one to the adjacent one, for example by means of welding points or lines, or other types of suitable mechanical connection, for example by gluing. Alternatively, the cutting members 12 are not welded or glued to each other but they can be retained together by an external retaining mean I which can comprise, or consist of, for example, a casing of plastic material which envelops them.

The present invention also concerns a method to produce a surgical tool 10 as described here.

In particular, the method comprises, at least, the following steps:
- producing, or having available, a plurality of cutting elements 12, each one distinct and separate from the other;
- disposing the cutting members 12 each one in contact with the adjacent one according to a determinate sequence along a common longitudinal axis X in order to make up a cutting unit 11.

According to one embodiment, the production of the cutting members 12 provides to make a central cavity 16 in each one of them.

In these cases, the method can also comprise a step of producing, or making available, a central support member 18 mating in shape with the central cavities 16 and the step of disposing the cutting members 12 in sequence is carried out by directly inserting each cutting member 12 on the central support member 18.

Alternatively, the step of disposing the cutting members 12 in sequence can be carried out with the aid of an external template mating in shape with the central cavities 16, and in which the cutting members 12 are inserted in sequence in order to make up the cutting unit 11, which will be subsequently removed from the template and inserted on the central support member 18.

In some embodiments, the method also comprises a connection step in which the cutting members 12 disposed in sequence are mechanically connected each one with the adjacent one. The connection is preferably made by welding but other types of suitable mechanical connection, such as gluing, are not excluded.

Alternatively, the connection step can provide to envelop the cutting unit 11 in a retaining mean I, which can be a casing of plastic material configured to retain them together.

It should be noted that the connection step as above can be carried out both when the cutting members 12 are disposed in sequence with the aid of the external template as above, and also when the cutting members 12 are disposed in sequence inserted directly on the central support member 18.

The production of the plurality of cutting members 12 and/or of the central support member 18 is preferably carried out by means of the three-dimensional laser cutting of a metal plate, for example of steel or titanium, having a thickness S. This is particularly advantageous because it considerably reduces the times, and consequently the costs, required to produce the cutting unit 11, especially when compared with the times required by conventional machining with removal of chip. Furthermore, this allows to customize the profile of the cutting unit 11 resulting from the sequence of the cutting members 12, modifying their shape as required.

Alternatively, the production of the plurality of cutting members 12 and/or of the central support member 18 can be carried out by means of one of the following processes: wire-cut EDM, milling, punching or coining of a metal plate, or by means of additive manufacturing technologies such as, for example, 3D printing of metals.

In addition, the method can provide the following steps:
- constraining the cutting unit 11 to the central support member 18 by means of a first element 19 disposed at a first end 22 of the central support member 18 and a second element 20 disposed at a second end 23 of the central support member 18.

It is clear that modifications and/or additions of parts or steps may be made to the surgical tool 10 and to the method as described heretofore, without departing from the scope of the present invention as defined by the claims.

In the following claims, the sole purpose of the references in brackets is to facilitate reading and they must not be considered as restrictive factors with regard to the field of protection defined by the claims.

## Claims

1. Surgical tool (10) configured to prepare a bone canal, comprising at least one cutting unit (11) configured to remove and/or compact bone tissues, wherein said cutting unit (11) comprises a plurality of autonomous cutting members (12) independent from each other and disposed each one in contact with the adjacent one according to a determinate sequence along a common longitudinal axis (X), wherein each of said cutting members (12) comprises a central cavity (16) substantially coaxial to said longitudinal axis (X) and that the plurality of central cavities (16) of said cutting members (12) define a central channel (17), wherein the central cavity (16) of each cutting member (12) is mating in shape with the next, or previous, cutting member (12) along the longitudinal axis (X), in order to give the central channel (17) a desired shape, the central channel (17) comprising in particular a first tapered portion (17a) and a second rectilinear portion (17b), wherein said surgical tool (10) also comprises a central support member (18) disposed inside said central channel (17) and configured to give structural rigidity to said cutting unit (11), wherein each cutting member (12) substantially comprises, or consists of, a metal plate having a determinate thickness (S) and has lateral walls inclined in the direction of the thickness (S) by an angle α with respect to the longitudinal axis (X), wherein said angle α is variable depending on the considered side of the cutting member (12) and it is comprised between about 10° and about 40°.

2. Surgical tool (10) as in claim 1, **characterized in that** it also comprises a first element (19) associated with said cutting unit (11) and configured to be mechanically connected to an external tool holder.

3. Surgical tool (10) as in one of the claims 1 or 2, **characterized in that** the cutting unit (11), as a whole, is oblong in shape and is substantially tapered along a longitudinal axis (X) thereof, from a first end (11a) to a second end (11b).

4. Surgical tool (10) as in claim 2 or as in claim 3 when combined with claim 2, **characterized in that** said first element (19) is connected to a first end (22) of said central support member (18) **and in that** a second element (20) is connected to a second end (23) of said central support member (18), opposite said first end (22).

5. Surgical tool (10) as in claim 4, **characterized in that** at least one of either said first element (19) or said second element (20) is made in a single body with said central support member (18).

6. Surgical tool (10) as in claim 4 or 5, **characterized in that** said first element (19) comprises a connector configured to be connected to an external tool holder.

7. Surgical tool (10) as in claim 4, 5 or 6 when combined with claim 3, **characterized in that** the cutting member (12) at the first end (11a) of the cutting unit (11) is mechanically connected, by welding or gluing, to the first element (19).

8. Surgical tool (10) as in claim 4, 5 or 6 when combined with claim 3, **characterized in that** the first element (19) is made in a single body with said central support member (18), and it is disposed at a first end (22) thereof.

9. Surgical tool (10) as in any claim from 4 to 8, **characterized in that** said second element (20), in cooperation with the first element (19), prevents the cutting unit (11) from sliding out of the central support member (18).

10. Surgical tool (10) as in any claim from 4 to 9, **characterized in that** the second end (23) of the central support member (18) comprises a coupling portion configured to be mechanically and reversibly connected to the second element (20), in such a way as to allow to disconnect the second element (20) from the central support member (18), allowing to replace the cutting unit (11).

11. Surgical tool (10) as in any claim from 4 to 9, **characterized in that** the second element (20) is made in a single body with the central support member (18).

12. Surgical tool (10) as in any claim from 4 to 11, **characterized in that** the first element (19) is mechanically and reversibly connected to the first end (22) of the central support member (18), in particular by means of a threaded coupling.

13. Surgical tool (10) as in any claim from 1 to 11, **characterized in that** said central support member (18) is mating in shape with the central channel (17), said central support member (18) comprising a first tapered portion (18a) and a second rectilinear portion (18b).

14. Surgical tool (10) as in any claim from 1 to 11, **characterized in that** the cross-section of the central cavities (16) and of the central support member (18) is quadrilateral, so as to prevent the rotation of the cutting members (12) around the longitudinal axis (X).

15. Surgical tool (10) as in any claim hereinbefore, **characterized in that** each of said cutting members (12) is connected mechanically, or by welding, with another adjacent one of said cutting members (12).

16. Surgical tool (10) as in any claim hereinbefore, **characterized in that** the cutting members (12) have a quadrilateral shape, or an elliptical, polygonal, or round shape, or other.

17. Surgical tool (10) as in any claim hereinbefore, **characterized in that** each cutting member (12), viewed in plan, has a determinate height (H) and a determinate width (W), wherein the cutting members (12) have a different height (H) and width (W) from each other and the sequence in which they are disposed along the longitudinal axis (X) is such as to give the cutting unit (11) a desired tapered profile.

18. Surgical tool (10) as in any claim hereinbefore, **characterized in that** each of the cutting members (12) also comprises at least one cutting element (13).

19. Surgical tool (10) as in claim 18, **characterized in that** the cutting elements (13) of each cutting member (12) comprise one or more lateral edges (14).

20. Surgical tool (10) as in claim 18, **characterized in that** the cutting elements (13) comprise a plurality of protrusions (15) made on the lateral walls of each cutting member (12), or, alternatively, in correspondence with a lateral edge (14).

21. Method to produce a surgical tool (10) configured to prepare a bone canal, the method comprising at least the following steps:
- producing, or having available, a plurality of cutting members (12), each one distinct and separate from the other; and
- disposing said cutting members (12) each one in contact with the adjacent one according to a determinate sequence along a longitudinal axis (X) in order to make up a cutting unit (11),
wherein each of said cutting members (12) comprises a central cavity (16) substantially coaxial to said longitudinal axis (X) and that the plurality of central cavities (16) of said cutting members (12) define a central channel (17), wherein the central cavity (16) of each cutting member (12) is mating in shape with the next, or previous, cutting member (12) along the longitudinal axis (X), in order to give the central channel (17) a desired shape, the central channel (17) comprising in particular a first tapered portion (17a) and a second rectilinear portion (17b), wherein said surgical tool (10) also comprises a central support member (18) disposed inside said central channel (17) and configured to give structural rigidity to said cutting unit (11), wherein each cutting member (12) substantially comprises, or consists of, a metal plate having a determinate thickness (S) and has lateral walls inclined in the direction of the thickness (S) by an angle α with respect to the longitudinal axis (X), wherein said angle α is variable depending on the considered side of the cutting member (12) and it is comprised between about 10° and about 40°.

22. Method as in claim 21, **characterized in that** it also comprises the following steps:
- producing, or having available, said central support member (18) mating in shape with said central channel (17); and
- inserting each of said cutting members (12) on said central support member (18) in accordance with said determinate sequence.

23. Method as in claim 21 or 22, **characterized in that** said cutting members (12) are made by means of one of the following processes: laser cutting, wire-cut EDM, milling, punching, coining of a metal plate; or by means of additive manufacturing technologies.

## Patentansprüche

1. Chirurgisches Instrument (10) zur Vorbereitung eines Knochenkanals, mit mindestens einer Schneideinheit (11) zum Entfernen und/oder Verdichten von Knochengewebe, wobei die Schneideinheit (11) mehrere voneinander unabhängige autonome Schneidelemente (12) umfasst, die jeweils in Kontakt mit dem benachbarten Element gemäß einer bestimmten Reihenfolge entlang einer gemeinsamen Längsachse (X) angeordnet sind, wobei jedes der Schneidelemente (12) einen zentralen Hohlraum (16) aufweist, der im Wesentlichen koaxial zur Längsachse (X) ist, und die mehreren zentralen Hohlräume (16) der Schneidelemente (12) einen zentralen Kanal (17) bilden, wobei der zentrale Hohlraum (16) jedes Schneidelements (12) in seiner Form mit dem nächsten oder vorherigen Schneidelement (12) entlang der Längsachse (X) zusammenpasst, um dem zentralen Kanal (17) eine gewünschte Form zu verleihen, wobei der zentrale Kanal (17) insbesondere einen ersten sich verjüngenden Abschnitt (17a) und einen zweiten geradlinigen Abschnitt (17b) umfasst, wobei das chirurgische Instrument (10) außerdem ein zentrales Stützelement (18) umfasst, das innerhalb des zentralen Kanals (17) angeordnet und so ausgebildet ist, dass es der Schneideinheit (11) strukturelle Steifigkeit verleiht, wobei jedes Schneidelement (12) im Wesentlichen eine Metallplatte mit einer bestimmten Dicke (S) umfasst oder daraus besteht und Seitenwände aufweist, die in Richtung der Dicke (S) unter einem Winkel α in Bezug auf die Längsachse (X) geneigt sind, wobei der Winkel α je nach der betrachteten Seite des Schneidelements (12) variabel ist und zwischen etwa 10° und etwa 40° liegt.

2. Chirurgisches Instrument (10) nach Anspruch 1, **dadurch gekennzeichnet, dass** es außerdem ein erstes Element (19) umfasst, das der genannten Schneideinheit (11) zugeordnet und so ausgebildet ist, dass es mechanisch mit einem externen Werkzeughalter verbunden werden kann.

3. Chirurgisches Instrument (10) nach einem der Ansprüche 1 oder 2, **dadurch gekennzeichnet, dass** die Schneideinheit (11) als Ganzes eine längliche Form aufweist und sich entlang ihrer Längsachse (X) von einem ersten Ende (11a) zu einem zweiten Ende (11b) im Wesentlichen verjüngt.

4. Chirurgisches Instrument (10) nach Anspruch 2 oder nach Anspruch 3 in Kombination mit Anspruch 2, **dadurch gekennzeichnet, dass** das erste Element (19) mit einem ersten Ende (22) des zentralen Stützelements (18) verbunden ist und dass ein zweites Element (20) mit einem zweiten Ende (23) des zentralen Stützelements (18) verbunden ist, das dem ersten Ende (22) gegenüberliegt.

5. Chirurgisches Instrument (10) nach Anspruch 4, **dadurch gekennzeichnet, dass** mindestens eines von dem ersten Element (19) und dem zweiten Element (20) einstückig mit dem zentralen Stützelement (18) ausgebildet ist.

6. Chirurgisches Instrument (10) nach Anspruch 4 oder 5, **dadurch gekennzeichnet, dass** das erste Element (19) ein Verbindungsstück umfasst, das so ausgebildet ist, dass es mit einem externen Werkzeughalter verbunden werden kann.

7. Chirurgisches Instrument (10) nach Anspruch 4, 5 oder 6 in Kombination mit Anspruch 3, **dadurch gekennzeichnet, dass** das Schneidelement (12) am ersten Ende (11a) der Schneideinheit (11) durch Schweißen oder Kleben mechanisch mit dem ersten Element (19) verbunden ist.

8. Chirurgisches Instrument (10) nach Anspruch 4, 5 oder 6 in Kombination mit Anspruch 3, **dadurch gekennzeichnet, dass** das erste Element (19) einstückig mit dem zentralen Stützelement (18) ausgebildet ist und an dessen erstem Ende (22) angeordnet ist.

9. Chirurgisches Instrument (10) nach einem der Ansprüche 4 bis 8, **dadurch gekennzeichnet, dass** das zweite Element (20) in Zusammenwirkung mit dem ersten Element (19) verhindert, dass die Schneideinheit (11) aus dem zentralen Stützelement (18) herausgleitet.

10. Chirurgisches Instrument (10) nach einem der Ansprüche 4 bis 9, **dadurch gekennzeichnet, dass** das zweite Ende (23) des zentralen Stützelements (18) einen Kupplungsabschnitt umfasst, der so ausgebildet ist, dass er mechanisch und reversibel mit dem zweiten Element (20) verbunden werden kann, und zwar in einer Weise, die es ermöglicht, das zweite Element (20) vom zentralen Stützelement (18) zu trennen, wodurch ein Austausch der Schneideinheit (11) ermöglicht wird.

11. Chirurgisches Instrument (10) nach einem der Ansprüche 4 bis 9, **dadurch gekennzeichnet, dass** das zweite Element (20) einstückig mit dem zentralen Stützelement (18) ausgebildet ist.

12. Chirurgisches Instrument (10) nach einem der Ansprüche 4 bis 11, **dadurch gekennzeichnet, dass** das erste Element (19) mechanisch und reversibel mit dem ersten Ende (22) des zentralen Stützelements (18) verbunden ist, insbesondere mittels einer Gewindeverbindung.

13. Chirurgisches Instrument (10) nach einem der Ansprüche 1 bis 11, **dadurch gekennzeichnet, dass** das zentrale Stützelement (18) in seiner Form an den zentralen Kanal (17) angepasst ist, wobei das zentrale Stützelement (18) einen ersten sich verjüngenden Abschnitt (18a) und einen zweiten geradlinigen Abschnitt (18b) umfasst.

14. Chirurgisches Instrument (10) nach einem der Ansprüche 1 bis 11, **dadurch gekennzeichnet, dass** der Querschnitt der zentralen Hohlräume (16) und des zentralen Stützelements (18) viereckig ist, um eine Drehung der Schneidelemente (12) um die Längsachse (X) zu verhindern.

15. Chirurgisches Instrument (10) nach einem der vorstehenden Ansprüche, **dadurch gekennzeichnet, dass** jedes der Schneidelemente (12) mechanisch oder durch Schweißen mit einem anderen benachbarten Schneidelement (12) verbunden ist.

16. Chirurgisches Instrument (10) nach einem der vorstehenden Ansprüche, **dadurch gekennzeichnet, dass** die Schneidelemente (12) eine viereckige, elliptische, polygonale, runde oder andere Form aufweisen.

17. Chirurgisches Instrument (10) nach einem der vorstehenden Ansprüche, **dadurch gekennzeichnet, dass** jedes Schneidelement (12), in der Draufsicht betrachtet, eine bestimmte Höhe (H) und eine bestimmte Breite (W) aufweist, wobei die Schneidelemente (12) untereinander unterschiedliche Höhen (H) und Breiten (W) aufweisen und die Reihenfolge, in der sie entlang der Längsachse (X) angeordnet sind, so gewählt ist, dass sie der Schneideinheit (11) ein gewünschtes sich verjüngendes Profil verleihen.

18. Chirurgisches Instrument (10) nach einem der vorstehenden Ansprüche, **dadurch gekennzeichnet, dass** jedes der Schneidelemente (12) zudem mindestens ein Schneideteil (13) umfasst.

19. Chirurgisches Instrument (10) nach Anspruch 18, **dadurch gekennzeichnet, dass** die Schneideteile (13) jedes Schneidelements (12) eine oder mehrere Seitenkanten (14) umfassen.

20. Chirurgisches Instrument (10) nach Anspruch 18, **dadurch gekennzeichnet, dass** die Schneideteile (13) eine Vielzahl von Vorsprüngen (15) aufweisen, die an den Seitenwänden jedes Schneidelements (12) oder alternativ im Bereich einer Seitenkante (14) ausgebildet sind.

21. Verfahren zur Herstellung eines chirurgischen Instruments (10), das zur Vorbereitung eines Knochenkanals ausgelegt ist, wobei das Verfahren mindestens die folgenden Schritte umfasst:
- Herstellen oder Bereitstellen einer Vielzahl von Schneidelementen (12), von denen jedes vom anderen verschieden und getrennt ist; und
- Anordnen der Schneidelemente (12), wobei jedes mit dem benachbarten in Kontakt steht, gemäß einer bestimmten Reihenfolge entlang einer Längsachse (X), um eine Schneideinheit (11) zu bilden,
wobei jedes der Schneidelemente (12) einen zentralen Hohlraum (16) aufweist, der im Wesentlichen koaxial zu der Längsachse (X) ist, und wobei die Vielzahl von zentralen Hohlräumen (16) der Schneidelemente (12) einen zentralen Kanal (17) bilden, wobei der zentrale Hohlraum (16) jedes Schneidelements (12) in seiner Form mit dem nächsten oder vorherigen Schneidelement (12) entlang der Längsachse (X) zusammenpasst, um dem zentralen Kanal (17) eine gewünschte Form zu verleihen, wobei der zentrale Kanal (17) insbesondere einen ersten sich verjüngenden Abschnitt (17a) und einen zweiten geradlinigen Abschnitt (17b) umfasst, wobei das chirurgische Instrument (10) außerdem ein zentrales Stützelement (18) umfasst, das innerhalb des zentralen Kanals (17) angeordnet und so ausgebildet ist, dass es der Schneideinheit (11) strukturelle Steifigkeit verleiht, wobei jedes Schneidelement (12) im Wesentlichen eine Metallplatte mit einer bestimmten Dicke (S) umfasst oder daraus besteht und Seitenwände aufweist, die in Richtung der Dicke (S) unter einem Winkel α in Bezug auf die Längsachse (X) geneigt sind, wobei der Winkel α je nach der betrachteten Seite des Schneidelements (12) variabel ist und zwischen etwa 10° und etwa 40° liegt.

22. Verfahren nach Anspruch 21, **dadurch gekennzeichnet, dass** es außerdem die folgenden Schritte umfasst:
- Herstellen oder Bereitstellen des zentralen Stützelements (18), das in seiner Form an den zentralen Kanal (17) angepasst ist; und
- Aufsetzen jedes der Schneidelemente (12) auf das zentrale Stützelement (18) gemäß der bestimmten Reihenfolge.

23. Verfahren nach Anspruch 21 oder 22, **dadurch gekennzeichnet, dass** die Schneidelemente (12) mittels eines der folgenden Verfahren hergestellt werden: Laserschneiden, Drahterodieren, Fräsen, Stanzen, Prägen einer Metallplatte; oder mittels additiver Fertigungstechnologien.

## Revendications

1. Instrument chirurgical (10) configuré pour préparer un canal osseux, comprenant au moins une unité de coupe (11) configurée pour enlever et/ou compacter des tissus osseux, dans lequel ladite unité de coupe (11) comprend une pluralité d'organes de coupe (12) autonomes, indépendants les uns des autres et disposés chacun en contact avec l'organe adjacent selon une séquence déterminée le long d'un axe longitudinal commun (X), dans lequel chaque organe de coupe (12) comprend une cavité centrale (16) sensiblement coaxiale audit axe longitudinal (X) et la pluralité de cavités centrales (16) desdits organes de coupe (12) définissant un canal central (17), la cavité centrale (16) de chaque organe de coupe (12) étant de forme complémentaire avec l'organe de coupe (12) suivant, ou précédent, le long de l'axe longitudinal (X), de manière à conférer au canal central (17) une forme souhaitée, le canal central (17) comprenant en particulier une première portion conique (17a) et une seconde portion rectiligne (17b), lequel instrument chirurgical (10) comprend également un organe central de support (18) disposé à l'intérieur dudit canal central (17) et configuré pour conférer une rigidité structurelle à ladite unité de coupe (11), chaque organe de coupe (12) comprenant sensiblement, ou consistant en, une plaque métallique ayant une épaisseur (S) déterminée et présentant des parois latérales inclinées dans la direction de l'épaisseur (S) selon un angle α par rapport à l'axe longitudinal (X), ledit angle α étant variable en fonction du côté considéré de l'organe de coupe (12) et étant compris entre environ 10° et environ 40°.

2. Instrument chirurgical (10) selon la revendication 1, **caractérisé en ce qu'**il comprend également un premier élément (19) associé à ladite unité de coupe (11) et configuré pour être relié mécaniquement à un porte-outil externe.

3. Instrument chirurgical (10) selon l'une quelconque des revendications 1 ou 2, **caractérisé en ce que** l'unité de coupe (11), dans son ensemble, est de forme oblongue et est sensiblement conique le long de son axe longitudinal (X), d'une première extrémité (11a) à une seconde extrémité (11b).

4. Instrument chirurgical (10) selon la revendication 2 ou selon la revendication 3 lorsqu'elle est combinée avec la revendication 2, **caractérisé en ce que** ledit premier élément (19) est relié à une première extrémité (22) dudit organe central de support (18) et **en ce qu'**un second élément (20) est relié à une seconde extrémité (23) dudit organe central de support (18), opposée à ladite première extrémité (22).

5. Instrument chirurgical (10) selon la revendication 4, **caractérisé en ce qu'**au moins l'un dudit premier élément (19) ou dudit second élément (20) est réalisé en corps monobloc avec ledit organe central de support (18).

6. Instrument chirurgical (10) selon la revendication 4 ou 5, **caractérisé en ce que** ledit premier élément (19) comprend un connecteur configuré pour être relié à un porte-outil externe.

7. Instrument chirurgical (10) selon la revendication 4, 5 ou 6 lorsqu'elle est combinée avec la revendication 3, **caractérisé en ce que** l'organe de coupe (12) à la première extrémité (11a) de l'unité de coupe (11) est relié mécaniquement, par soudage ou collage, au premier élément (19).

8. Instrument chirurgical (10) selon la revendication 4, 5 ou 6 lorsqu'elle est combinée avec la revendication 3, **caractérisé en ce que** le premier élément (19) est réalisé en corps monobloc avec ledit organe central de support (18), et est disposé à une première extrémité (22) de celui-ci.

9. Instrument chirurgical (10) selon l'une quelconque des revendications 4 à 8, **caractérisé en ce que** ledit second élément (20), en coopération avec le premier élément (19), empêche l'unité de coupe (11) de sortir par glissement de l'organe central de support (18).

10. Instrument chirurgical (10) selon l'une quelconque des revendications 4 à 9, **caractérisé en ce que** la seconde extrémité (23) de l'organe central de support (18) comprend une portion d'accouplement configurée pour être reliée mécaniquement et de manière réversible au second élément (20), de façon à permettre de déconnecter le second élément (20) de l'organe central de support (18), permettant ainsi de remplacer l'unité de coupe (11).

11. Instrument chirurgical (10) selon l'une quelconque des revendications 4 à 9, **caractérisé en ce que** le second élément (20) est réalisé en corps monobloc avec l'organe central de support (18).

12. Instrument chirurgical (10) selon l'une quelconque des revendications 4 à 11, **caractérisé en ce que** le premier élément (19) est relié mécaniquement et de manière réversible à la première extrémité (22) de l'organe central de support (18), en particulier au moyen d'un accouplement fileté.

13. Instrument chirurgical (10) selon l'une quelconque des revendications 1 à 11, **caractérisé en ce que** ledit organe central de support (18) est de forme complémentaire avec le canal central (17), ledit organe central de support (18) comprenant une première portion conique (18a) et une seconde portion rectiligne (18b).

14. Instrument chirurgical (10) selon l'une quelconque des revendications 1 à 11, **caractérisé en ce que** la section transversale des cavités centrales (16) et de l'organe central de support (18) est quadrilatère, de manière à empêcher la rotation des organes de coupe (12) autour de l'axe longitudinal (X).

15. Instrument chirurgical (10) selon l'une quelconque des revendications précédentes, **caractérisé en ce que** chacun desdits organes de coupe (12) est relié mécaniquement, ou par soudage, à un autre organe de coupe (12) adjacent.

16. Instrument chirurgical (10) selon l'une quelconque des revendications précédentes, **caractérisé en ce que** les organes de coupe (12) présentent une forme quadrilatère, ou une forme elliptique, polygonale, circulaire, ou autre.

17. Instrument chirurgical (10) selon l'une quelconque des revendications précédentes, **caractérisé en ce que** chaque organe de coupe (12), vu en plan, présente une hauteur (H) déterminée et une largeur (W) déterminée, les organes de coupe (12) ayant une hauteur (H) et une largeur (W) différentes les uns des autres et la séquence selon laquelle ils sont disposés le long de l'axe longitudinal (X) étant telle qu'elle confère à l'unité de coupe (11) un profil conique souhaité.

18. Instrument chirurgical (10) selon l'une quelconque des revendications précédentes, **caractérisé en ce que** chacun des organes de coupe (12) comprend également au moins un élément de coupe (13).

19. Instrument chirurgical (10) selon la revendication 18, **caractérisé en ce que** les éléments de coupe (13) de chaque organe de coupe (12) comprennent une ou plusieurs arêtes latérales (14).

20. Instrument chirurgical (10) selon la revendication 18, **caractérisé en ce que** les éléments de coupe (13) comprennent une pluralité de saillies (15) réalisées sur les parois latérales de chaque organe de coupe (12), ou, alternativement, au niveau d'une arête latérale (14).

21. Procédé pour produire un instrument chirurgical (10) configuré pour préparer un canal osseux, le procédé comprenant au moins les étapes suivantes :
- produire, ou disposer, une pluralité d'organes de coupe (12), chacun distinct et séparé des autres ; et
- disposer lesdits organes de coupe (12) chacun en contact avec l'organe adjacent selon une séquence déterminée le long d'un axe longitudinal (X) afin de constituer une unité de coupe (11),
lequel procédé est tel que chacun desdits organes de coupe (12) comprend une cavité centrale (16) sensiblement coaxiale audit axe longitudinal (X) et que la pluralité de cavités centrales (16) desdits organes de coupe (12) définit un canal central (17), la cavité centrale (16) de chaque organe de coupe (12) étant de forme complémentaire avec l'organe de coupe (12) suivant, ou précédent, le long de l'axe longitudinal (X), de manière à conférer au canal central (17) une forme souhaitée, le canal central (17) comprenant en particulier une première portion conique (17a) et une seconde portion rectiligne (17b), lequel instrument chirurgical (10) comprend également un organe central de support (18) disposé à l'intérieur dudit canal central (17) et configuré pour conférer une rigidité structurelle à ladite unité de coupe (11), chaque organe de coupe (12) comprenant sensiblement, ou consistant en, une plaque métallique ayant une épaisseur (S) déterminée et présentant des parois latérales inclinées dans la direction de l'épaisseur (S) selon un angle α par rapport à l'axe longitudinal (X), ledit angle α étant variable en fonction du côté considéré de l'organe de coupe (12) et étant compris entre environ 10° et environ 40°.

22. Procédé selon la revendication 21, **caractérisé en ce qu'**il comprend également les étapes suivantes:
- produire, ou disposer, ledit organe central de support (18) de forme complémentaire audit canal central (17); et
- insérer chacun desdits organes de coupe (12) sur ledit organe central de support (18) conformément à ladite séquence déterminée.

23. Procédé selon la revendication 21 ou 22, **caractérisé en ce que** lesdits organes de coupe (12) sont réalisés au moyen de l'un des procédés suivants : découpe laser, découpe par fil EDM, fraisage, poinçonnage, matriçage d'une plaque métallique ; ou au moyen de technologies de fabrication additive.
